# EUROPEAN PATENT APPLICATION

(11) **EP 2 478 911 A1**
(43) Date of publication of application: **25.07.2012**
(21) Application number: 12151176.0
(22) Date of filing: 24.11.2009
(51) Int. Cl.: A61K 35/76, A61K 39/395

(54) **Method for treating Parkinson's disease using filamentous bacteriophage**

(30) Priority: 24.11.2008 US 11744608 P
(62) Divisional of application: 09775018.6
(71) Applicant: Ramot at Tel Aviv University Ltd., 61392 Tel Aviv (IL)
(72) Inventor: Solomon, Beka, 46399 Herzliya Pituach Herzliya (IL); Dimant, Haim, Bat Yam 59446 (IL)
(74) Representative: Algemeen Octrooi- en Merkenbureau

(57) **Abstract**

The present invention relates to the use of a filamentous bacteriophage that does not display (i) a mammalian cell internalization signal; (ii) an α-synuclein antigen or α-synuclein antibody; or (iii) a β-amyloid antigen or β-amyloid antibody, either alone or in combination with a filamentous bacteriophage which displays an antibody that specifically binds to a pro-inflammatory cytokine, to treat Parkinson's disease.

## Description

### BACKGROUND OF THE INVENTION

### Field of the Invention

The invention relates to therapeutics and methods for treating Parkinson's disease.

### Description of the Related Art

Parkinson's disease (PD) is a progressive neurodegenerative disease whose primary clinical features include motor abnormalities, such as resting tremor, bradykinesia and rigidity (Fahn and Sulzer, 2004). PD is characterized by the loss of dopaminergic neurons in the substantia nigra pars compacta and the presence of inclusion bodies, called Lewy bodies and Lewy neurites, in the surviving neurons of the same region (Forno, 1996). Although it is generally accepted that the loss of midbrain dopaminergic neurons is largely responsible for the major motor symptoms, this is not the only region showing pathologic changes in PD patients. Lewy pathology and cell loss first appear in lower brain stem nuclei, progressively ascend to the midbrain and finally to cortical areas in a highly predictable manner (Braak et al., 2004). Progression of the Lewy pathology to the various regions outside the midbrain may account for the abundance of the secondary symptoms commonly observed in PD patients, such as depression, dementia, and various autonomic and sensory dysfunctions.

Although the cause of PD remains elusive, there is a large body of evidence suggesting that misfolding and abnormal aggregation of α-synuclein is an important component of the disease pathogenesis. Genetic linkage analyses have identified three missense mutations in the inherited forms of parkinsonism (Kruger et al. 1998; Polymeropoulos et al. 1997; Zarranz et al. 2004), and all the mutant variants have been shown to accelerate either oligomerization or fibrillation (Conway et al. 2000; Greenbaum et al. 2005). Accumulation of wild type α-synuclein is sufficient to cause the disease.

Fibrillar aggregates of α-synuclein seem to be the main component of Lewy bodies and Lewy neurites, and these are now considered the most reliable PD marker for postmortem diagnosis (Spillantini et al. 1998). Because α-synuclein is a cytosolic protein it has been assumed that the pathogenic changes and effects induced by the protein occur in the cytoplasm and are limited to the single cell. However, recent studies of extracellular α-synuclein suggest that the scope of pathogenic action goes beyond the cytoplasm of its origin (Lee, 2008).

The presence of α-synuclein and its aggregated forms in extracellular fluid was recently demonstrated both *in vivo* and *in vitro.* Extracellular α-synuclein appears to be delivered by unconventional exocytosis of intravesicular α-synuclein, although the exact mechanism has not been characterized. Intravesicular α-synuclein is prone to aggregation and is the potential source of extracellular aggregates.

The role of secreted α-synuclein in the extracellular space can be inferred from studies using tissue culture systems. Several studies reported cytotoxic effects of extracellular α-synuclein and its internal hydrophobic fragment (nonamyloid component or NAC) when the proteins were added to the culture medium (Albani et al. 2004; Bodles et al. 2000; Du et al. 2003; El-Agnaf et al. 1998; Forloni et al. 2000; Lee et al. 2004; Seo et al. 2002; Sung et al. 2001). Some studies have demonstrated the toxic effect of fibrillar aggregates (Bodles et al. 2000; El-Agnaf et al. 1998), while other studies identified protofibrillar or oligomeric aggregates as the toxic culprit (Du et al. 2003).

Alpha synuclein can readily incorporate into membranes and can be found in synaptic vesicles and on the cell membrane. There are not many well-structured models for the mechanisms of toxicity. Recent studies illustrate a possible role for an α-synuclein pore-like protofibrils in the pathogenesis of Parkinson's disease (Tsigelny et al., 2007; Lee et al., 2002; Volles and Lansbury, 2002). One model proposes that oligomeric α-synuclein can form annular structures with a central pore (Volles and Lansbury 2003). These aggregates can bind to membranes (Volles et al. 2001) and their membrane permeabilizing action has been demonstrated in synthetic model membranes, such as phospholipid liposomes (Volles et al. 2001) and planar bilayer membranes (Kayed et al. 2004). Insertion of these aggregates into the cell membrane would have a catastrophic effect on cell viability due to the free exchange of ions and small metabolites between the cytoplasm and the extracellular space. Although this toxic pore model explains the cytotoxicity of at least some oligomeric aggregates, the pores and the pore activity have yet to be demonstrated in biological systems. Another potential mechanism of neurotoxicity of extracellular α-synuclein, and especially its aggregate forms, may involve neuroinflammatory responses (Zhang et al., 2005; Klegeris et al., 2006).

Despite the advances made to date in developing therapies to treat PD, there is still a great need for additional therapies.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention provides a filamentous bacteriophage for use in treating Parkinson's disease or susceptibility to Parkinson's disease, and a method for treating a patient suffering from or susceptible to Parkinson's disease (PD). The method involves administering to the patient a filamentous bacteriophage which does not display a mammalian cell internalization signal.

The present invention also provides a pharmaceutical composition containing a filamentous bacteriophage displaying an antibody specific to a pro-inflammatory cytokine and a second filamentous bacteriophage which does not display (i) a mammalian cell internalization signal; (ii) an α-synuclein antigen or α-synuclein antibody; (iii) a β-amyloid antigen or β-amyloid antibody; or (iv) an antibody specific to a pro-inflammatory cytokine.

### BRIEF DESCRIPTION OF THE DRAWINGS

Figures 1A and 1B show a computer model of membrane α-synuclein (AS) aggregate with a perspective view from the front of an α-synuclein aggregate embedded in the cell membrane in a pore like structure (Fig. 1A) and a cross-sectional view of the membrane, showing the depth of the protein insertion into the membrane (Fig. 1 B) (Tsigelny et al., 2007).

Figures 2A and 2B show the disaggregating activity of phage on AS fragment aggregates *in vitro* as measured by Tht (Fig 2A) and visualized by TEM (Fig 2B).

Figure 3 is a graph depicting the effects of phage on viability of SH-SY5Y cells.

Figures 4A and 4B show the reduction of AS aggregates by phage (helper) as visualized (Fig 4A) and measured in an ELISA (Fig 4B) using an α-synuclein polyclonal antibody in an α-synuclein filter retardation assay.

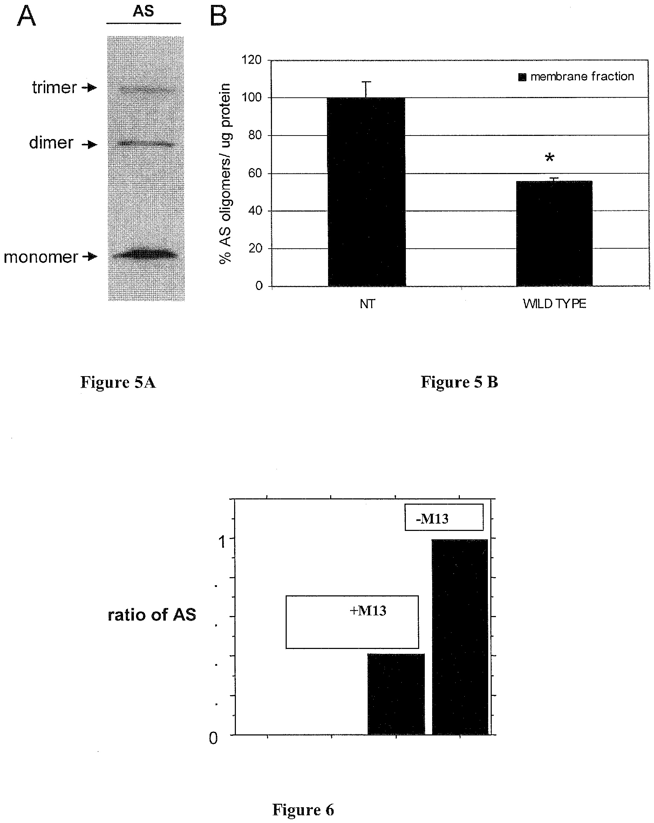

Figure 5A shows Western blot analysis of the membrane fraction of SH-SY5Y cells, demonstrating the presence of various α-synuclein (AS) oligomers, and Figure 5B shows ELISA assay for measurement of oligomers after M13 treatment. In Fig. 5A, AS is detected with a polyclonal antibody against AS (Sigma). In Fig. 5B, the amount of AS oligomers from the membrane fraction was quantified in an ELISA specific for AS oligomers which are detected with a monoclonal antibody against AS (Sigma, clone Syn 211). A significant reduction in the amount of AS oligomers was measured in the membrane fraction of SH-SY5Y cells treated with wild type filamentous phages. *= significance difference compared to non treated cells (p<0.05).

Figure 6 is a graph showing reduction in AS reactivity after interaction with M13 phages compared to vehicle. The data is expressed as a ratio of the average number of neuronal aggregates of AS observed in one hemisphere of the brain injected with M13 compared to the other hemisphere injected with PBS vehicle (+M13). In the -M13 control, both hemispheres were injected with PBS vehicle.

### DETAILED DESCRIPTION OF THE INVENTION

### Definitions

For purposes of this specification and the accompanying claims, the following definitions apply.

The terms "patient", "subject" and "recipient" are used interchangeably. They include humans and other mammals which are the object of therapeutic treatment.

The term "treating" with respect to Parkinson's disease is intended to mean substantially inhibiting, slowing or reversing the progression of Parkinson's disease, such as reducing or inhibiting the formation of aggregates of α-synuclein, or disaggregating pre-formed aggregates of α-synuclein; substantially ameliorating one or more clinical symptoms of Parkinson's disease, such as reducing inflammation associated with Parkinson's disease; or substantially preventing the appearance of clinical symptoms of Parkinson's disease.

The term "co-administer" is intended to mean administration by means of a single dosage form or by means of multiple dosage forms administered simultaneously, sequentially or separately. Preferably, co-administration causes the effects of each administration to be exerted on the cells being treated at an overlapping period of time, more preferably simultaneously.

The term "antibody" as used herein includes polyclonal antibodies, monoclonal antibodies, antibody compositions with polyepitope specificities, bispecific antibodies, diabodies, or other purified preparations of antibodies and recombinant antibodies. The antibodies can be whole antibodies, e.g., of any isotype (IgG, IgA, IgE, IgM, etc.), or antibody fragments that bind the antigen of interest. In a specific example of an antibody used in the present invention, the antibody to be formulated is an antibody having the IgG isotype. Antibodies can be fragmented using conventional or other techniques and the fragments screened for binding to an antigen of interest. Generally, an antibody fragment comprises the antigen-binding and/or the variable region of an intact antibody.

The term "antibody fragment" includes segments of proteolytically cleaved or recombinantly prepared portions of an antibody molecule that can selectively bind to a selected protein. Non-limiting examples of such proteolytic and/or recombinant fragments include Fab, F(ab')2, Fab', Fv, and single chain antibodies (scFv) containing a V_{L} and/or V_{H} domain joined by a peptide linker, domain antibodies (dAbs), Nanobodies® (antibody-derived biological therapeutic agents that contain the unique structural and functional properties of naturally-occurring heavy-chain antibodies), and UniBodies (antibodies lacking the hinge region). The scFvs may be covalently or noncovalently linked to form antibodies having two or more binding sites.

In some embodiments, the antibody or antibody fragment is a humanized monoclonal antibody or fully humanized monoclonal antibody. The term "humanized monoclonal antibody" as used herein is a monoclonal antibody from a non-human source (recipient) that has been altered to contain at least one or more of the amino acid residues found in the equivalent human monoclonal antibody (donor). A "fully humanized monoclonal antibody" is a monoclonal antibody from a non-human source that has been altered to contain all of the amino acid residues found in the antigen-binding region of the equivalent human monoclonal antibody. Humanized antibodies may also comprise residues that are not found either in the recipient antibody or the donor antibody. These modifications can be made to further refine and optimize antibody functionality. A humanized antibody may also optionally comprise at least a portion of a human immunoglobulin constant region (Fc).

The term "pro-inflammatory cytokine" refers to any proinflammatory cytokine involved in brain inflammation associated with Parkinson's disease, which preferably includes IL-6, IL-1, IL-17 and TNFα, and is most preferably IL-6.

The term "mammalian cell internalization signal" refers to any cell adhesion sequence which facilitates internalization as a result of cell adhesion/attachment to the cell. Numerous mammalian cell adhesion sequences are known and include the Arg-Gly-Asp (RGD) cell adhesion sequence, the Tat peptide from HIV and peptides comprising the sequence of Arg-Glu-Asp (RED), Arg-Lys-Lys (RKK), Leu-Asp-Val (LDV; Humphries, 1992), Leu-Leu-Gly (LLG; Koivunen et al., 2001), Asp-Gly-Glu-Ala (DGEA; SEQ ID NO:2), lle-Arg-Val-Val-Met (IRVVM; SEQ ID NO:3; Kosfeld et al., 1993), Pro-His-Ser-Arg-Asp (PHSRN; SEQ ID NO:4) and RFYVVMWK (SEQ ID NO:5; Kosfeld et al., 1993). Many cell adhesion sequences (also known as cell attachment motifs) are known in cell adhesive molecules such as laminin, fibronectin, vitronectin, fibrinogen, thrombospondin, etc.

The term "α-synuclein antigen" refers to an antigen from human α-synuclein, where the human α-synuclein preferably has the amino acid sequence of SEQ ID NO:6 (NCBI gene ID:6622, accession no. NP000336; UniProtKB/Swiss-Prot accession no. P37840). An "α-synuclein antibody" is one which recognizes and binds to the α-synuclein SEQ ID NO:6 or a variant or mutant thereof.

The term "β-amyloid antigen" refers to an antigen from a plaque forming "β-amyloid peptide", also known as "βAP", "βA", "Aβ" or "AβP", derived from human amyloid precursor protein. A "β-amyloid antibody" is one which recognizes and binds to the β-amyloid peptide of naturally occurring human A β1-42 peptide and variants and mutants thereof.

As used herein, a "pharmaceutical composition" refers to a preparation of one or more of the active ingredients described herein with other chemical components such as physiologically suitable carriers and excipients. The purpose of a pharmaceutical composition is to facilitate administration of a compound to a patient.

The phrases "physiologically acceptable carrier" and "pharmaceutically acceptable carrier," which may be interchangeably used, refer to a carrier or a diluent that does not cause significant irritation to an organism and does not abrogate the biological activity and properties of the administered compound.

The term "excipient" refers to an inert substance added to a pharmaceutical composition to further facilitate administration of an active ingredient. Non-limiting examples of excipients include calcium carbonate, calcium phosphate, various sugars and types of starch, cellulose derivatives, gelatin, vegetable oils and polyethylene glycols.

The term "wild-type filamentous bacteriophage" as used herein means a naturally occurring filamentous bacteriophage that is isolated away from other components with which it is typically associated in nature. The term also includes commercially available filamentous phage that are characterized as "wild-type".

The term "inactivated wild-type filamentous bacteriophage" as used herein means a wild-type filamentous bacteriophage that is not genetically altered by recombinant DNA means, but has been rendered incapable of replication, such as by UV-irradiation. Any mechanism which renders the phage incapable of replication, but does not disturb the filamentous structure of the bacteriophage (retains its ability to penetrate into the brain through the olfactory pathway) is contemplated by this invention.

The term "WT phage" refers to both wild-type filamentous bacteriophage and inactivated wild-type filamentous bacteriophage.

### Filamentous Bacteriophage for Use in Treatment and Methods of Treatment

In one embodiment, the invention provides a filamentous bacteriophage for use in treating Parkinson's disease or susceptibility to Parkinson's disease and a method of treating a patient suffering from or susceptible to Parkinson's disease by administering to the patient a filamentous bacteriophage, wherein the bacteriophage does not display (i) a mammalian cell internalization signal, (ii) an α-synuclein antigen or α-synuclein antibody, or (iii) a β-amyloid antigen or β-amyloid antibody.

In one aspect of this embodiment, the bacteriophage is administered to the patient as part of a pharmaceutically acceptable composition additionally comprising a pharmaceutically acceptable carrier.

In another aspect of this embodiment, the bacteriophage is a WT phage.

Without being bound to theory, it is proposed that the phage utilized in this invention bind to extracellular α-synuclein or α-synuclein aggregates in the membrane and reduce the aggregation of α-synuclein in the cell membrane. The present inventors propose that this reduction of α-synuclein aggregates in the membrane also leads to a reduction of intracellular aggregates of α-synuclein, possibly by shifting the equilibrium of α-synuclein from intracellular to extracellular. Nevertheless, without being bound to any particular mechanism, the present filamentous bacteriophage and method are useful for treating a patient suffering from or susceptible to Parkinson's disease.

In one embodiment, the filamentous bacteriophage is useful for intranasal administration. Intranasal administration allows these bacteriophage to cross the blood-brain barrier. The phage are then eliminated from the brain and body via urine and feces without adverse effects on peripheral organs.

Pro-inflammatory cytokines contribute to the neuroinflammatory symptoms of Parkinson's disease. Removal of such pro-inflammatory cytokines would ameliorate such symptoms. Thus in one embodiment, the invention provides a filamentous bacteriophage displaying an antibody specific to a pro-inflammatory cytokine for use in treating Parkinson's disease or susceptibility to Parkinson's disease and a method of treating a patient suffering from or susceptible to Parkinson's disease by administering to the patient a filamentous bacteriophage displaying an antibody specific to a pro-inflammatory cytokine. In one aspect of this embodiment, the bacteriophage is administered intranasally. In another aspect, the bacteriophage is administered as part of a pharmaceutically acceptable composition additionally comprising a pharmaceutically acceptable carrier. In another aspect, the antibody specific to a pro-inflammatory cytokine is of the IgG class and bears an Fc portion. In still another aspect the antibody is an antibody specific for IL-6. In yet another aspect the bacteriophage bearing the pro-inflammatory cytokine does not comprise a mammalian cell internalization signal.

When administered intranasally, the antibody-bearing phage will be delivered to the brain where they will be directed to the pro-inflammatory cytokines by the antibodies displayed thereon, thereby inactivating such cytokines. The phage portion of these phage-displayed antibodies will have the dual action of getting the antibodies past the blood-brain barrier and directly inhibiting α-synuclein aggregation.

Accordingly to a related embodiment, the invention provides a first and second filamentous bacteriophage for use in treating and a method of treating a patient suffering from or susceptible to Parkinson's disease by co-administering to the patient (a) a first filamentous bacteriophage displaying an antibody specific to a pro-inflammatory cytokine; and (b) a second filamentous bacteriophage, wherein the second bacteriophage does not display an antibody specific to a pro-inflammatory cytokine. In one aspect, the second filamentous phage additionally does not display a mammalian cell internalization signal. In another aspect, the second filamentous phage additionally does not display (i) an α-synuclein antigen or α-synuclein antibody; or (ii) a β-amyloid antigen or β-amyloid antibody. In still another aspect, the second filamentous phage additionally does not display (i) a mammalian cell internalization signal; (ii) an α-synucleinantigen or α-synuclein antibody; or (iii) a β-amyloid antigen or β-amyloid antibody.

In another aspect, each filamentous bacteriophage is administered as part of a pharmaceutically acceptable composition additionally comprising a pharmaceutically acceptable carrier. In another aspect of any of the compositions set forth above, the second filamentous bacteriophage is a WT phage. In still another aspect, the second filamentous bacteriophage is a UV-irradiated bacteriophage.

In yet another aspect, the first and the second bacteriophage are each administered to the patient intranasally. In another aspect, the antibody specific to a pro-inflammatory cytokine is of the IgG class and bears an Fc portion. In still another aspect, the antibody is an antibody specific for IL-6. In another aspect, the first bacteriophage does not comprise a mammalian cell internalization signal.

Phages can be made to display the antibodies to pro-inflammatory cytokines by any technique known to the art. For example, the antibody can be engineered as a single-chain antibody by well-known techniques and the phage vector can be modified so as to display such single-chain antibodies (scFv) on the bacteriophage surface.

Another way to cause any given antibody, preferably a monoclonal antibody, to be displayed on a phage is to produce a phage that displays a polypeptide that binds the Fc portion of immunoglobulins. Such phage are known in the art and described in PCT publication WO2007/095616. Using such a modified phage, any antibody containing an Fc portion can be caused to be displayed thereon by simply contacting the antibodies with the phage. The Fc portion of the antibody will be bound by the Fc-binding polypeptide displayed by the phage. Thus, the binding of an antibody, or fragment thereof, is facilitated.

In one embodiment a polypeptide that binds the Fc portion of immunoglobulins is protein A, protein G, a fragment thereof containing the antibody binding portion of protein A (i.e., binding domain B) or protein G, or a variant of protein A or protein G. In another embodiment, the protein A variant is the variant having the amino acid sequence of SEQ ID NO:1. When an antibody lacking an Fc region is used, it must be directly displayed by the phage.

Filamentous bacteriophages are a group of structurally related viruses which contain a circular single-stranded DNA genome. They do not kill their host during productive infection. The phages that infect *Escherichia coli* containing the F plasmids are collectively referred to as Ff bacteriophages. They do not infect mammalian cells. In one embodiment, each filamentous bacteriophage used in the methods set forth above is independently selected from filamentous phages M13, f1, and fd. In one aspect, when a first and a second filamentous phage are used, each phage is of the same subtype and is selected from filamentous phages M13, f1, and fd.

Phage that bear either an antibody specific to a pro-inflammatory cytokine or a polypeptide that binds the Fc portion of immunoglobulins are engineered to display the protein on its surface. This typically involves the expression of a cDNA clone of the polypeptide to be displayed, as a fusion protein with a phage coat protein. Filamentous bacteriophages that display foreign proteins or peptides as a fusion with a phage coat protein are well known to those in the art. A variety of phages and coat proteins may be used, including, but not limited to: M13 protein III, M13 protein VIII, M13 protein VI, M13 protein IX, and fd minor coat protein pIII (Saggio et al., 1995; Uppala and Koivunen, 2000). A large array of vectors are available to produce such fusion proteins (see Kay et al., 1996; Berdichevsky et al., 1999; and Benhar, 2001). Methods for inserting foreign coding sequences into a phage gene are well known (see e.g., Sambrook et al., 1989; and Brent et al., 2003).

In one embodiment, a polypeptide that binds the Fc portion of immunoglobulins is displayed by its fusion to the minor coat protein (protein III) of a filamentous phage.

Methods delineated herein also include those wherein the patient is identified as in need of a particular stated treatment. Identifying a patient in need of such treatment can be in the judgment of a patient or a health care professional and can be subjective (e.g. opinion) or objective (e.g. measurable by a test or diagnostic method).

### Pharmaceutical Compositions

In a related embodiment, the invention provides a pharmaceutical composition (e.g., pyrogen-free) comprising: (a) a first filamentous bacteriophage displaying an antibody specific to a pro-inflammatory cytokine; and (b) a second filamentous bacteriophage, wherein the second bacteriophage does not display an antibody specific to a pro-inflammatory cytokine. In one aspect, the second filamentous phage additionally does not display a mammalian cell internalization signal. In another aspect, the second filamentous phage additionally does not display (i) an α-synuclein antigen or α-synuclein antibody; or (ii) a β-amyloid antigen or β-amyloid antibody. In still another aspect, the second filamentous phage additionally does not display (i) a mammalian cell internalization signal; (ii) an α-synuclein antigen or α-synuclein antibody; or (iii) a β-amyloid antigen or β-amyloid antibody.

In one aspect of this embodiment, the second bacteriophage is a WT phage. In a more specific aspect, the second bacteriophage is a UV-irradiated bacteriophage.

In another aspect, the antibody specific to a pro-inflammatory cytokine is of the IgG class and bears an Fc portion. In yet another aspect, the first bacteriophage in the composition displays an antibody specific to IL-6. In still another aspect, the first bacteriophage does not comprise a mammalian cell internalization signal.

In a further aspect, the composition is formulated for intranasal administration.

Techniques for formulation and administration of drugs may be found in "Remington's Pharmaceutical Sciences," Mack Publishing Co., Easton, PA, latest edition, which is incorporated herein by reference and are well known in the art.

Pharmaceutical compositions for use in accordance with the present invention thus may be formulated in a conventional manner using one or more physiologically acceptable carriers comprising excipients and auxiliaries, which facilitate processing of the active ingredients into preparations which can be used pharmaceutically.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. A nasal spray, which does not require a pressurized pack or nebulizer as in an inhalation spray, can alternatively used for intranasal administration. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

Pharmaceutical compositions suitable for use in the context of the method of the present invention include compositions wherein the active ingredient(s) is contained in an amount effective to achieve the intended purpose. More specifically, an effective amount means an amount of active ingredient(s) effective to treat Parkinson's disease.

Determination of a therapeutically effective amount is well within the capability of those skilled in the art, especially in light of the detailed disclosure provided herein.

Dosage amount and interval may be adjusted individually to provide brain levels of the filamentous virus display vehicle which are sufficient to treat (minimal effective concentration, MEC). The MEC will vary for each preparation, but can be estimated from *in vitro* data. Dosages necessary to achieve the MEC will depend on individual characteristics.

Dosage intervals can also be determined using the MEC value. Preparations should be administered using a regimen, which maintains brain levels above the MEC for 10-90% of the time, preferably between 30-90% of the time and most preferably between 50-90% of the time during the course of treatment.

Depending on the severity and responsiveness of Parkinson's disease to be treated in the patient, dosing can be of a single or a plurality of administrations, with the course of treatment lasting from several days to several weeks or until diminution of the Parkinson's disease state is achieved.

The amount of a composition to be administered will, of course, be dependent on the subject being treated, the severity of the affliction, the judgment of the prescribing physician, etc.

Compositions used in the method of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as if further detailed above.

In one particular embodiment, the invention provides a kit to treat Parkinson's disease comprising, in separate containers, (a) a first filamentous bacteriophage displaying an antibody specific to a pro-inflammatory cytokine; and (b) a second filamentous bacteriophage, wherein the second bacteriophage does not display (i) a mammalian cell internalization signal; (ii) an α-synuclein antigen or α-synuclein antibody; (iii) a β-amyloid antigen or β-amyloid antibody; or (iv) an antibody specific to a pro-inflammatory cytokine; and (c) instructions describing a method of using the kit to treat Parkinson's disease.

Having now generally described the invention, the same will be more readily understood through reference to the following example which is provided by way of illustration and is not intended to be limiting of the present invention.

### EXAMPLE

The experimental results in this example demonstrate the effect of filamentous phage on disaggregation of α-synuclein aggregates involved in the pathogenesis of PD.

### Preparation of filamentous phages

M13 filamentous phages were prepared from infected TG1 *Escherichia* coli cultures with M13K07 helper phage (New England Biolabs, Beverly, MA) in 2YT broth containing 50 µg/ml kanamycin (Sigma-Aldrich, St. Louis, MO). Bacterial cells were centrifuged (8300×g, 15 min), and phages were precipitated from the supernatant by addition of 1/5 (wt/vol) of 16.7% polyethylene glycol (PEG) and centrifuged (14,000×g, 1 h at 4°C). The pellet was resuspended in sterile phosphate-buffered saline (PBS) (3% of the supernatant volume). Residual bacteria were removed by centrifugation at 6,000×g for 15 min, and the phages then subjected to PEG-NaCl precipitation. The pellet was finally resuspended in PBS (2% of the supernatant volume) and phage solution filtered through a pyrogen-free 0.45 µm filter to remove any residual bacteria. Spherical phages were generated by incubation of filamentous phages in PBS with an equal volume of chloroform. The solution was vortexed six times each for 10 s, over 3 min at room temperature (RT) and centrifuged at 18,5000xg for 1 min. Both aqueous and chloroform solutions were left uncovered in the hood until all chloroform residues had evaporated and then resuspended in PBS to the original volume. Phage integrity was disrupted by sonicating 500 µl of 1 x 10¹³ phages in PBS on ice for 15 x 5 sec using ultrasonic cell disruptor (Microson Heat Systems, Farmingdale, NY, USA).

### Investigation of α-synuclein aggregation in vitro

The level of aggregation of both a 12 amino acid fragment corresponding to amino acids 71-82 of the human α-synuclein (SEQ ID NO:7), the core of Lewy bodies, and the complete recombinant alpha-synuclein protein were tested and analyzed.

The ability of filamentous phage to prevent and disaggregate both the peptide and the recombinant alpha-synuclein was examined. The level of α-synuclein aggregation was determined on the Thioflavine T (Tht) and protein filtration assays as described below.

### Alpha-synuclein fibrils and filamentous phages were visualized by electron microscopy.

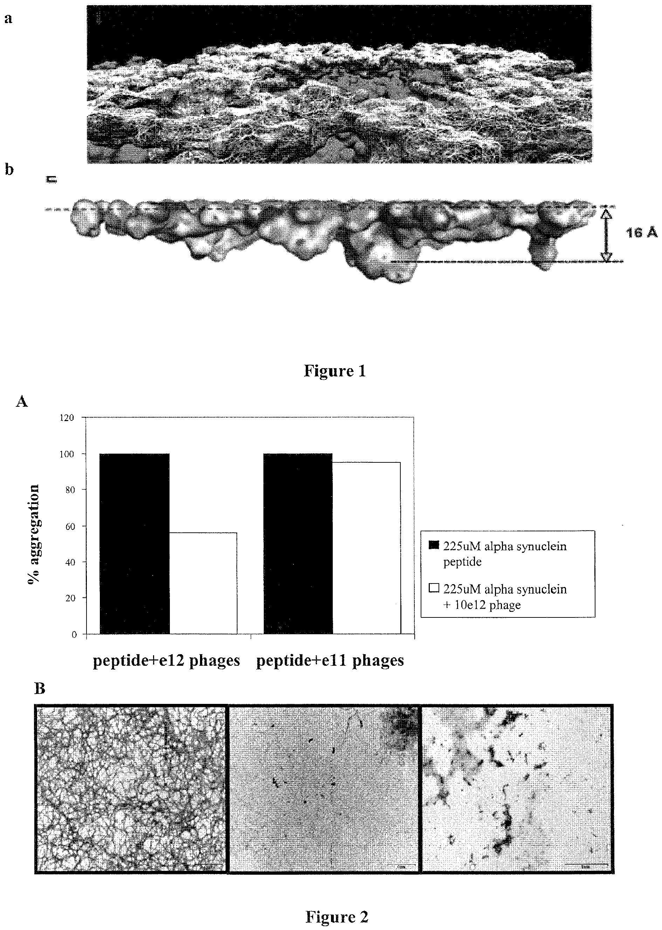

In order to evaluate the anti-aggregating effect of the filamentous phage, 900 µl samples of α-synuclein fragment spanning amino acid residues 71-82 of α-synuclein were incubated for six weeks at 37°C. At this stage, filamentous phages were incubated with the aggregated peptide for a period of one week. Two concentrations of phages were examined: 10¹²/ml and 10¹¹/ml. Peptide samples were diluted into an aqueous 0.3 µM Tht solution to a final concentration of 225 µM. Samples fluorescence emission at 480 nm was quantified. A significant 43% reduction was detected in the sample containing the amount of 10¹²/ml phages as viewed by a reduction in emission at 480 nm (Fig. 2A).

Samples were also viewed by transmission electron microscopy (TEM). Samples were loaded on carbon grids and were coated with uranyl acetate. In the sample containing only the α-synuclein peptide, significant amounts of complex fibrils in a dense form were detected (Fig. 2B, left panel). A substantial reduction in the amount and complexity of fibrils was viewed in the sample incubated with 10¹¹ phages, while in the sample incubated with 10¹² phages, no fibrils were detected and only amorphous aggregates were visible (Fig. 2B, middle and right panels).

### SH-S Y5Y ce// cultures as cellular model

The neuroblastoma cell line SH-SY5Y is a good candidate to use as PD model since it is a dopaminergic cell. The cells were stably transfected with the wild type human α-synuclein gene and the mutant α-synuclein A53T gene.
- alpha-synuclein expression was determined by Western blot.
- cells were stained with alpha-synuclein antibody to visualize Lewy bodies.

SH-SY5Y cells stably transfected to over-express A53T α-synuclein were grown in 10 cm dishes and maintained in DMEM:Ham's F12 (1:1) modified medium containing 1% non-essential amino-acids (NEAAs) and supplemented with FCS (10%), glutamine (2 mM), penicillin-streptomycin solution (1%)(Biological Industries), in humidified incubator at 37°C with 5% CO₂.

### Cell lysis

For separation of α-synuclein, the lysis procedure was performed using the cell extraction described by Lee et al. (2004) as modified by the laboratory of the present inventors. Briefly, cells were rinsed with PBS, trypsinized with 1.5ml trypsin per dish collected to a 15ml tube, centrifuged and washed again with PBS followed by collection to an Eppendorf tube. Cells were lysed with 100µl buffer T (containing 20mM Tris pH7.4, 25mM KCI, 5mM MgCl₂, 0.25M sucrose, 1% Triton X-100 and protease inhibitor mixture), pipetted until no cell clumps remained, incubated at room temperature for 10 min. and centrifuged at 13,000 kg for 10 min. Supernatant was collected and placed in a separate Eppendorf tube marked as "sup" (note that no supernatant should be left with the pellet fraction). The pellet with gently covered with buffer N (containing 0.1M Na₂CO₃, pH 11.5 and a protein inhibitor mixture) without mixing and incubated overnight at 4°C. This allowed the proteins to gently resuspend in the buffer. Following the incubation, the now cloudy buffer was removed using a small pipette tip and discarded. The remaining material in the tube was marked as "pellet". The supernatant and pellet extracts were kept at -20°C and analyzed by Western blot to quantitate the amount of soluble and insoluble α-synuclein.

### Phage toxicity

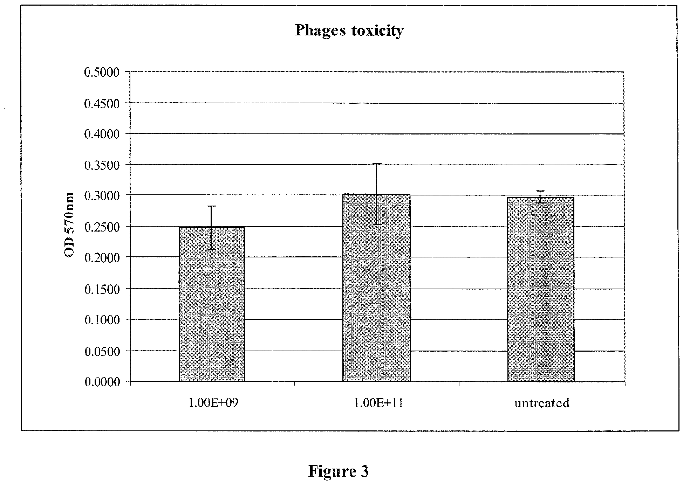

Viability of differentiated SH-SY5Y cells following incubation with phage was tested using the MTT reagent. Cells were incubated overnight with 1x10⁹ and 1x10¹¹ phages/well in a 96 well plate. No cell death was observed following the incubation (Fig. 3).

### Filter retardation assay

For protein aggregate filtration, a commercially available slot blot device (Bio-Rad Laboratories, Munich, Germany) was used. Samples were filtered through a blocked nitrocellulose membrane (0-.2 µm pore size, Schleicher & Scheull, Dassel, Germany). After filtration, each slot was washed with 0.1 % SDS. Alpha-synuclein aggregates were detected using the monoclonal antibody LB509 (1:10,000) with a secondary HRP-coupled goat-anti-mouse antibody and finally visualized by chemiluminescence. Densitometric quantification of PAF blot was performed with SigmaGel v1.0.

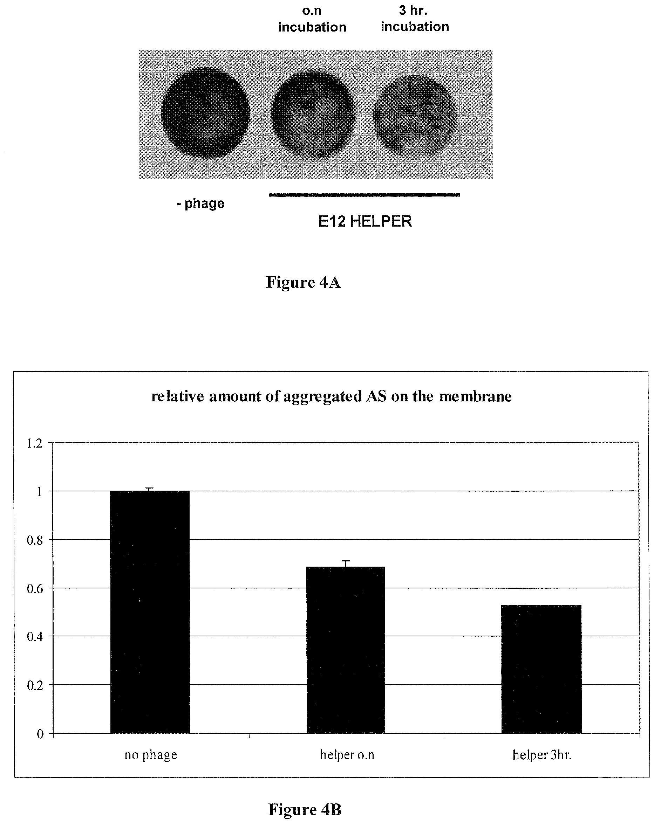

Equal numbers of cells per 10 cm dish were added and allowed to reach 80% confluency. At that time, cells were treated with different amounts of phage for 24 and 72 hr. Cells were then collected from each plate and lysed and analysed, as described below. Differentiated SH-SY5Y cells overexpressing wild type α-synuclein were incubated with a total of 1x10¹² phages for a period of 3hr. or overnight at 37°C. The soluble and insoluble fractions were extracted and the insoluble fraction was filtered through a 0.2µm nitrocellulose membrane. The amount of α-synuclein aggregates retained on the filter was higher in the untreated cells (Fig. 4A).

### ELISA of α-synuclein oligomers

An ELISA to detect oligomeric species of α-synuclein (El-Agnaf et al., 2000) was modified in order to measure the amount of α-synuclein oligomers in the insoluble fraction of SH-SY5Y cells. In order to detect only oligomers of AS, the same monoclonal antibody employed for coating (conjugated to biotin) was used.

A 40%-50% reduction in AS oligomers was detected in the insoluble fraction of cells treated with phage both 3hr. and overnight incubation (Fig. 4B).

### Interaction of M13 phage with α-synuclein in cellular models

Alpha-synuclein (AS) is a natively unfolded protein localized at presynaptic terminals. AS readily incorporates into membranes through the N-terminus and thus exists in the cell in two forms: a membrane bound and a disordered cytosolic form. Accumulating data indicate that the membrane form of AS plays a role in cellular toxicity. Protofibrils can form annular structures embedded on the cell membrane and cause membrane permeability. It was recently demonstrated that AS has a propensity for higher aggregation in the presence of lipids and that the membrane aggregates can induce aggregation of the cytosolic form of AS.

Here, the modulation of AS oligomers following treatment with wild-type (M13) filamentous phages in SH-SY5Y cells over-expressing wild-type AS is presented. The membrane fraction of the cells were extracted using a membrane extraction kit (MBL) for AS detection by Western blot analysis. Not only were AS monomers detected, but AS dimers and trimers were also detected in the membrane fraction (Fig. 5A), demonstrating the existence of AS oligomers in membrane compartments in our cellular model. The effect of filamentous phage on oligomerization of the AS membrane following incubation with filamentous phages is shown in Figs. 5A and 5B. SH-SY5Y cells were differentiated using retinoic acid and were incubated overnight with wild-type phages at 1⁰¹¹ phages/ ml. The membrane fractions were extracted the following day and samples of each extraction were measured for the amount of AS oligomers in an ELISA designed to recognize only oligomeric species of AS (Fig. 5B). A significant reduction in AS oligomers from the membrane fraction was measured in SH-SY5Y cells following incubation with filamentous phages. The effect of wild-type phages on AS aggregation extracted from the membrane fraction suggests that the wild-type phages affect AS on the plasma membrane, possibly via direct interaction with AS through a previously demonstrated region of interaction located at amino acids 73-85 of the NAC region of AS. It is also possible that due to the long incubation of the cells with phages, a small percentage of the phages are internalized into the cells affecting other membranous compartments in the cell other than the plasma membrane. Binding of the phages to the cells with possible internalization may promote clearance of alpha-synuclein from the plasma membrane through activation of lysosmal degradation.

### Studies of M13 interaction with α-synuclein using transgenic mice model of Parkinson's disease

PDGF α-synuclein transgenic mice (D line) and non-transgenic mice aged 7 months received intra-hippocampal injections with M13 phage at 1x10¹⁴ and the brains were analyzed 7 days later by IHC with antibodies against alpha-synuclein, M13 and Iba1 (for microglia activation). Abundant M13 immunoreactivity was observed in alpha-synuclein Tg mice injected with M13. M13 immunostaining was observed in neuronal cell bodies in the neocortex and hippocampus. In the hippocampus M13 immunostaining was also abundant in the neuropil. The average number of neuronal aggregates of α-synuclein was measured in both hemispheres of the brain after M 13 injection in one hemisphere and phosphate buffered saline (PBS) vehicle in the other hemisphere, and the ratio between the measurements in the separate hemispheres is shown in Figure 6.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

Reference to known method steps, conventional methods steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### REFERENCES

Benhar et al., Phage display of single-chain antibodies. In: J. Colligen (Ed), Current Proteocols in Immunology, Vol. 10.19B, John Wiley & Sons, Inc, USA (2001)
Berdichevsky et al., J. Immunol. Methods, 228:151-62 (1999)
Brent et al., Current Protocols in Molecular Biology, John Wiley & Sons Inc. (2003)
El-Agnaf, 0 M, Jakes, R, Curran, M D, Middleton, D, Ingenito, R., Bianchi, E, et al. Aggregates from mutant and wild-type alpha-synuclein proteins and NAC peptide Induce apoptotic cell death In human neuroblastoma cells by formation of beta-sheet and amyloid-like filaments. FEBS Letters, 440:71-75 (1998).
El-Agnaf, O. M. A. et al., FASEB J. 20, 419-425 (March 1, 2006, 2006).
Fahn, S., & Sulzer, D. Neurodegeneration and neuroprotection in Parkinson disease NeuroRx:, 1:139-154 (2004).
Forloni, G., Bertani, I., Calella, A. M., Thaler, F., & Invernizzi, R. Alpha-synuclein and Parkinson's disease: Selective neurodegenerative effect of alpha-synuclein fragment on dopaminergic neurons in vitro and in vivo, Annals of Neurology, 47:632-640 (2000).
Forno, L. S. Neuropathology of Parkinson's disease. Journal of Neuropathology and Experimental Neurology, 55:259-272 (1996).
Greenbaum, E. A.. Graves, C. L., Mishlzen-Eberz, A. J., Lupoli, M. A., Lynch, D. R., Englander, S. W., et al. The E46K mutation In alpha-synuclein increases amyloid fibril formation. Journal of Biological Chemistry, 280: 7800-7807 (2005).
Humphries, Peptide recognition motifs involved in the binding of integrins to their ligands, Kidney International, 41:645-649 (1992)
Kay et al., Phage Display of Peptides and Proteins, A Laboratory Manual, Academic Press (1996)
Klegeris, A., Giasson, B. I., Zhang, H., Maguire, J., Pelech, S., & McGeer, P. L. Alpha-synuclein and its disease-causing mutants induce ICAM-1 and IL-6 in human astrocytes and astrocytoma cells. FASEB Journal, 20:2000-2008 (2006).
Koivunen et al., "Inhibition of β2 integrin-mediated leukocyte cell adhesion by leucine-leucine-glycine motif-containing peptides, The Journal of Cell Biology, 153(5):905-915 (2001)
Kosfeld et al., "Identification of a new cell adhesion motif in two homologous peptides from the COOH-terminal cell binding domain of human thrombospondin" The Journal of Biological Chemistry, 268(12):8808-8814 (1993)
Kruger, R., Kuhn, W., Muller, T., Woitalla, D., Graeber, M.. Kosel. S, et al. Ala30Pro mutation in the gene encoding alpha synuclein in Parkinson's disease. Nature Generics, 18:106-108 (1998).
Lee. E N , Cho, H. J , Lee, C. H., Lee, D , Chung, K. C , & Palk, S. R. Phthalocyanine tetrasulfonates affect the amyloid formation and cytotoxicity of alpha-synuclein. Biochemistry, 43:3704-3715 (2004).
Lee, H. J., C. Choi, S.-J. Lee, J. Biol. Chem. 277, 671-678 (2002).
Lee, S. J., Origins and effects of extracellular §-synuclein: Implications in Parkinson's Disease, J. Mol. Neurosci., 34:17-22 (2008)
Masliah et al., Effects of α-synuclein immunization in a mouse model of Parkinson's disease, Neuron, 46:857-868 (2005)
Polymeropoulos, M. H., Lavedan, C., Leroy, E., Ide, S. E., Dehejia, A, Dutra, A., et al. Mutation In the alpha-synuclein gene identified in families with Parkinson disease, Science, 276:2045-2047 (1997).
Saggio et al., Gene, 152:35-39 (1995)
Sambrook et al., Molecular cloning: A laboratory manual, Cold Spring Harbor Laboratory, Cold Spring Harbor, NY (1989)
Seo, J. ti.. Rah, J. C., Choi, S. H., Shin, J. K., Min, K,, Kim, H. S., et al. Alpha-synuclein regulates neuronal survival via Bcl-2 family expression and P13iAkt kinase pathway. MSEB Journal, 16:1826-1828 (2002).
Spillantini, M. G., Crowther, R. A., Jakes, R., Elasegawa, M., Goedert, M. alpha-synuclein in filamentous inclusions of Lewy bodies from Parkinson's disease and dementia with lewy bodies Proceedings of the National Academy of Sciences of the United States of America, 95:6469-6473 (1998).
Sung, J. Y., Kim, J. Palk, S. R., Park, J. H., Ahn, Y. S., & Chung, K. C. Induction of neuronal cell death by Rab5A-dependent endocytosis of alpha-synuclein. Journal of Biological Chemistry, 276:27441-27448 (2001).
Tsigelny et al., FEBS Journal 274, 1862-1877 (2007).
Uppala and Koivunen, Chem. High Throughput Screen, 3:373-392 (2000)
Volles, M. J., & Lansbury Jr., P. T. Zeroing In on the pathogenic form of alpha-synuclein and its mechanism of neurotoxcity Parkinson's disease. Biochemistry, 42:7871-7878 (2003).
Volles, M. J, Lee, S.-J., Rochet, J. C., Shtileman, M D., Ding, T. T., Kessler, J. C., et al. Vesicle permeabilization by protofibrillar alpha-synuclein: Implications for the pathogenesis and treatment of Parkinson's disease. Biochemistry, 40:7812-7819 (2003).
Volles, M. J., P. T. Lansbury, Biochemistry 41, 4595-4602 (2002).
Zarranz, J J., Alegre, J., Gomez-Esteban, J. C , Lezcano, E , Ros, R, Ampuero, I. et al. The new mutation, E46K, of alpha-synuclein causes Parkinson and Lewy body dementia Annals of Neurology, 55:164-173 (2004).
Zhang, W, Wang, T, Pei, Z., Miller, D. S, Wu, X., Block, M L., et al. Aggregated alpha-synuclein activates microglia: A process leading to disease progression in Parkinson's disease FASEB Journal, 19:533-542 (2005).

## Claims

1. A filamentous bacteriophage for use in treating Parkinson's disease or susceptibility to Parkinson's disease, wherein the filamentous bacteriophage does not display (i) a mammalian cell internalization signal; (ii) an α-synuclein antigen or α-synuclein antibody; or (iii) a β-amyloid antigen or β-amyloid antibody.

2. A first and second filamentous bacteriophage for use in treating Parkinson's disease, wherein:
a) the first filamentous bacteriophage displays an antibody to a pro-inflammatory cytokine; and
b) the second filamentous bacteriophage does not display (i) a mammalian cell internalization signal; (ii) an α-synuclein antigen or α-synuclein antibody; (iii) a β-amyloid antigen or β-amyloid antibody; or (iv) an antibody to a pro-inflammatory cytokine.

3. A pharmaceutical composition comprising:
a) a first filamentous bacteriophage displaying an antibody specific to a pro-inflammatory cytokine;
b) a second filamentous bacteriophage, wherein the second bacteriophage does not display (i) a mammalian cell internalization signal; (ii) an α-synuclein antigen or α-synuclein antibody; (iii) a β-amyloid antigen or β-amyloid antibody; or (iv) an antibody specific to a pro-inflammatory cytokine; and
c) a pharmaceutically acceptable carrier.

4. A composition for treating a patient suffering from or susceptible to Parkinson's disease, wherein the composition comprises a filamentous bacteriophage, wherein the bacteriophage does not display (i) a mammalian cell internalization signal; (ii) an α-synuclein antigen or α-synuclein antibody; or (iii) a β-amyloid antigen or β-amyloid antibody.

5. The filamentous phage of claim 2, or the composition of claim 3, wherein the bacteriophage displaying an antibody specific to a pro-inflammatory cytokine does not display a mammalian cell internalization signal.

6. The filamentous phage or composition of any one of claims 1 to 5, wherein the bacteriophage that does not display (i) a mammalian cell internalization signal; (ii) an α-synuclein antigen or α-synuclein antibody; or (iii) a β-amyloid antigen or β-amyloid antibody is a WT phage.

7. The filamentous phage or composition of claim 6, wherein the bacteriophage is a UV-irradiated phage.

8. The filamentous phage or composition of any one of claims 2, 3, or 5 to 7, wherein the filamentous bacteriophage displaying an antibody that binds to a pro-inflammatory cytokine further displays an antibody binding portion of protein A or protein G, and wherein the antibody that binds to a pro-inflammatory cytokine is bound to the antibody binding portion of protein A or protein G.

9. The filamentous phage or composition of any one of claims 2, 3, or 5 to 8, wherein the antibody that binds to a pro-inflammatory cytokine is an antibody that binds to IL-6.

10. The filamentous phage or composition of any one of claims 1 to 9, wherein each bacteriophage is administered intranasally.

11. The filamentous phage or composition of any one of claims 1 to 10, wherein each filamentous bacteriophage is independently selected from M13, f1, and fd bacteriophage, and mixtures thereof.

12. The filamentous phage or composition of claim 11, wherein each filamentous bacteriophage is M 13.

13. The filamentous phage or composition of any one of claims 1, or 4-12, wherein the bacteriophage that does not display (i) a mammalian cell internalization signal; (ii) an α-synuclein antigen or α-synucleinantibody; or (iii) a β-amyloid antigen or β-amyloid antibody, also does not display (iv) an antibody to a pro-inflammatory cytokine.
